# EUROPEAN PATENT APPLICATION

(11) **EP 1 226 816 A1**
(43) Date of publication of application: **31.07.2002**
(21) Application number: 02001479.1
(22) Date of filing: 22.01.2002
(51) Int. Cl.: A61K 7/48

(54) **Anti-wrinkle cosmetic product**

(30) Priority: 24.01.2001 IT PN010004
(71) Applicant: Granzotto, Federico, 33077 Sacile, PN (IT)
(72) Inventor: Granzotto, Federico, 33077 Sacile, PN (IT)
(74) Representative: Dalla Rosa, Adriano

(57) **Abstract**

Anti-wrinkle cosmetic product, to be applied on skin with wrinkles and bags to stretch and to make it smooth. Product comprising a composition formed by at least a tensor agent including a first component silica and a second component water, both acting as active principle, and also formed by or more excipients of the kinds commonly used in cosmetic industry.

## Description

The invention relates to an anti-wrinkle cosmetic product, fit to be applied on skin with wrinkles and bags to stretch and to make it smooth.

At present time, many cosmetic products are used for being applied particularly on face skin in which wrinkles and bags are present, in order to stretch it and make it smoother. However, these products do not eliminate completely wrinkles and bags, in that this effect can be obtained only with one or more surgical operations. The present invention relates to a new type of anti-wrinkle cosmetic product, which has a different composition with respect to the cosmetic products used hitherto, and includes a small number of components permitting to obtain an excellent and complete anti-wrinkle effect on the skin to which it is applied.

This cosmetic product is made by the composition hereinafter described, with particular reference to the accompanying patent claims. The invention will be better understood thanks to the following description of this cosmetic product.

The anti-wrinkle cosmetic product in accordance to the invention is composed of an anti-wrinkle tensor agent including the two main components silica and water acting as active principle, capable of stretching uniformly skin and making it smooth, which components can be also added by excipients which will be described.

In particular, silica is a pyrogenic amorphous silica normally acting as thickening agent but which has been surprisingly found fit to act as a tensor agent, namely an agent used for stretching skin and consequently used here for performing this function. Silica is formed by spherical particles having a diameter comprised between 7 to 40 nm, whose surface is constituted by groups of siloxanes (Si-O-Si-O-Si-) and silanoles (-Si-OH) with a larger amount of siloxanes groups, that make this compound an inert compound. Silica, when is surrounded by water molecules, as in the present combination of the two main components silica and water, forms a gelified structure. The so combined two components silica and water may be used as a sol solution or system, or a gel solution or system, or an aerosol solution or system.

The two main components can be combined and mixed together at ambient temperature, with a percentage of silica comprised preferably from 0,1 % to 3 % weight with respect to the weight of the resulting composition and a percentage of water of 99,9 % to 97 % weight with respect to the weight of the resulting composition, where water can be also added at smaller percentages when one or more excipients are added to this composition.

The present composition can be in case added to excipients performing particular functions and which can be for example of the following kinds :
a) moisturizing agents;
b) soothing agents;
c) moistening agents ;
d) emollient agents;
e) refreshing agents;
f) thickening agents;
g) preservative agents;
h) stabilizing agents;
i) film making agents.

Hereinafter, at least one possible agent and its percentage in weight which may be used for each excipient is described by way of an example.
a) moisturizting agent : Panthenol from 0.1 to 3 percent weight; Sodium Hyaluronate from 0.5 to 3 percent weight;
b) soothing agent : Etoxydiglycol, Caprylyl Glycol, Sodium Polyacrylate, each from 0.1 to 3 percent weight;
c) moistening agent : Glycerin, Propylenglycol, each from 10 to 30 percent weight;
d) emollient : Dimethicone Copolyol from 0.1 to 5 percent weight;
e) refreshing agent : Aloe Barbadensis from 0.5 to 3 percent weight;
f) thickening agent : Xanthan Gum from 0.1 to 3 percent weight;
g) preservative agent : as under b);
h) stabilizing agent : Algin from 0.1 to 3 percent weight;
i) film making agent : Agar from 0.1 to 3 percent weight.

Of course, also other excipients can be used, combined with different percentages weight with silica and water, thus without departing from the sphere of the present invention.

By way of an example only, other excipients which can be used are :
- Disodium Edta from 0.1 to 1 percent weight;
- Salicylic Acid from 0.1 to 0.5 percent weight;
- Sodium Alginate, Osmocide, etc...

The so prepared composition, when applied on skin, forms a homogeneous film and causes skin to absorb it and to be stretched and smoothed, thereby producing the tensor effect. Moreover, this composition thanks to the capacity of retaining water, provides a moisturizing effect on skin surface, with improvement of its aesthetical look (smoother and more uniform skin), and thanks to slightly acid pH it allows skin biological balance to be preserved.

## Claims

1. Anti-wrinkle cosmetic product, fit to be applied on skin and particularly on face skin, for obtaining an excellent and complete anti-wrinkle effect, so as to stretch skin and make it smoother, **characterized by** a composition formed by at least a tensor agent including a first component silica and a second component water, both acting as active principle, combined and mixed together preferably at ambient temperature, and also formed by one or more possible excipients combined and mixed to said silica and water preferably at ambient temperature.

2. Anti-wrinkle cosmetic product according to claim 1, **characterized in that** said first and second component may be used as a sol solution or system, or as a gel solution or system or as an aerosol solution or system.

3. Anti-wrinkle cosmetic product according to claim 2, **characterized in that** said silica and said water are used preferably with a percentage weight respectively of 0,1 % to 3 % and 99, 9 % to 97 %.

4. Anti-wrinkle cosmetic product according to claim 2, **characterized in that** said excipients are the same normally used in cosmetic products, such as for example moisturizing agents, soothing agents, moistening agents, emollient agents, refreshing agents, thickening agents, preservative agents, stabilizing agents, film making agents and the like.

5. Anti-wrinkle cosmetic product according to the previous claims, **characterized by** the use of silica as tensor agent forming a first component in a composition having water as a second component and also possible excipients of the types commonly used in cosmetic industry.
